(19) Europäisches Patentamt European Patent Office Office européen des brevets

(11) **EP 3 545 914 A1**

(12) **EUROPEAN PATENT APPLICATION**

(43) Date of publication:
**02.10.2019 Bulletin 2019/40**

(21) Application number: **19166427.5**

(22) Date of filing: **29.03.2019**

(51) Int Cl.:
**A61F 2/56** (2006.01)    **A61F 2/68** (2006.01)
**A61F 2/58** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **29.03.2018 GB 201805307**

(71) Applicant: **Open Bionics Ltd Bristol BS34 8QZ (GB)**

(72) Inventors:
• **GIBBARD, Joel**
**Backwell, Bristol BS48 3LE (GB)**
• **PAYNE, Samantha**
**Backwell, Bristol BS48 3LE (GB)**
• **RAINES, Jonathan**
**Horfield, Bristol BS7 0BN (GB)**
• **MCVEIGH, Brian**
**St Werburghs, Bristol BS2 9UD (GB)**
• **QATTAN, Ahlam**
**Barnoldswick, Wiltshire BB18 6AA (GB)**
• **WOOD, Steve**
**Swindon, Wiltshire SN2 1LT (GB)**

(74) Representative: **Bryers LLP**
**7 Gay Street**
**Bath, Bath and North East Somerset BA1 2PH (GB)**

(54) **IMPROVEMENTS IN OR RELATING TO PROSTHETIC LIMBS**

(57) A prosthetic digit is provided and comprises one or more channels through which tendon-like members can pass to cause flexing of a joint. The channel/s have a mouth at each end thereof, the mouths being rounded, whereby in use to spread the normal tendon force over a larger area, thereby reducing the pressure.

Figure 8

EP 3 545 914 A1

**Description**

The present invention relates generally to prosthetic limbs and particularly, although not exclusively, to a hand for a robotic prosthetic arm.

A prosthesis is an artificial device that replaces a missing body part, which may be lost through trauma, disease, or congenital conditions. Prosthetics are intended to restore the normal functions of the missing body part.

The present invention seeks to provide improvements in or relating to prosthetic limbs.

An aspect of the present invention provides a prosthetic digit comprising one or more channels through which tendon-like members can pass to cause flexing of a joint, the channel/s having a mouth at each end thereof, the mouths being rounded, whereby in use to spread the normal tendon force over a larger area, thereby reducing the pressure.

In some embodiment fingers may be flexed by means of tendons that run through tendon channels in the phalanges. In some embodiments the tendons are on the palmar side of the joints, the effect of the actuators retracting the tendons is therefore to flex the fingers. The magnitude of the flexing moment applied by the tendon at each joint is proportional to its perpendicular distance to it.

By moving the tendon away from the joint centre, a larger moment may be generated for the same tension in tendon. The tension is related to the strength of the actuator. As this is essentially a question of leverage, increasing the perpendicular distance also increases the distance a tendon has to be retracted to fully flex a joint. In some embodiments the actuators in the hand produce linear motion through a lead screw. They therefore have a limited amount of travel. Therefore, the tendon channels have to be carefully positioned so that the full travel of the motor is used.

When the joint is flexed, the tendon is bent through a large angle around a small radius, resulting in a very high normal force. This can produce a 'cheese-wire' effect leading to notches around the tendon channel mouth. The notches add friction and effectively change the perpendicular distance to the joint affecting travel. In order to prevent this the mouths of the tendon channel may be rounded, spreading the normal force over a larger area, reducing the pressure.

The profile of the curve assumed by the tendon may be an arc which is tangential to the palmar edge of the tendon channel and perpendicular to the joint face. The curves on either side of a joint also have the same radius. The perpendicularity and radius ensures the tendon is wrapped over a smooth (G 2 smooth) curve. The curvature also moves the tendon away from the joint as the finger flexes, increasing leverage. This helps to counteract the increasing extension moments caused by the ligament and the springs.

The digit may have phalanges and the channels pass through the phalanges.

The tendon may terminate in a tip region of the digit.

The digit may have means for adjusting the tension in the tendon.

The digit may have phalanges which can flex via retraction of a tendon-like member.

The finger may have, for example on its dorsal sides, an extension spring for causing the finger to extend when the tendon is relaxed.

The present invention also provides a prosthetic hand or foot having one or more digits as described herein.

The hand or foot may comprise one or more actuators for actuating digits.

The hand or foot may comprise a main control PCB.

A hand formed in accordance with the present invention may have four fingers and a thumb.

The present invention also provides a prosthetic finger comprising a channel through which a tendon-like member can pass to cause flexing of a joint, the channel having a mouth at each end thereof, the mouths being rounded.

In some embodiment more tendon is assigned to a proximal joint than to a distal joint, whereby the proximal joint flexes before the distal joint.

In some embodiment in use the tendon is positioned towards the top of the channel when the finger is relaxed, and moved to the bottom of the channel when the finger is bent.

In some embodiment, in a bent position the tendon is constantly curved in an arc across the joint.

A further aspect of the present invention provides a prosthetic hand having one or more digits, the hand comprising one or more actuators for actuating digits, and a main control PCB.

In some embodiments the hand has four fingers and a thumb.

The present invention also provides a prosthetic finger/thumb comprising one or more channels through which tendon-like members can pass to cause flexing of a finger joint, the channel/s having a mouth at each end thereof, the mouths being rounded, whereby in use to spread the normal tendon force over a larger area, reducing the pressure.

The digits may have phalanges, with the channels passing through the phalanges.

The present invention also provides a prosthetic finger/thumb having phalanges which can be caused to flex with respect to each other via retraction of a tendon-like member, the finger having, on its dorsal sides, an extension spring for causing the finger to extend when the tendon is relaxed.

The present invention also provides a prosthetic finger/thumb having a tendon for causing flex, the tendon terminating in a finger tip region, the finger having means for adjusting the tension in the tendon.

A prosthetic hand comprising one or more fingers/digits as described herein is also provided.

Also provided are: a prosthetic arm as described herein; a hand as described herein; a finger as described herein; and an actuator block as described herein.

A present invention also provides a robotic prosthetic arm, comprising a ventilated outer frame and a ventilated inner liner.

The present invention also provides a prosthesis, such as a prosthetic arm, comprising an outer frame and an inner socket, the outer frame having attachment points for receiving a removable cover.

According to a further aspect of the present invention there is provided a prosthetic arm comprising an outer frame and an inner socket, the outer frame having a plurality of airflow openings and the inner socket having a plurality of airflow openings.

In some embodiments the present invention provides a transradial prosthesis - an artificial limb that replaces an arm missing below the elbow. In other embodiments the present invention provides a transhumeral prosthesis - a prosthetic lower and upper arm, including a prosthetic elbow.

In some embodiments the present invention provides or relates to a myoelectric prosthesis, which uses the electrical tension generated every time a muscle contracts, as information.

The outer frame may have an open core lattice structure. This provides strength whilst at the same time inherently providing ventilation.

The socket may have a plurality of longitudinal flutes. The socket may, therefore, have a generally cylindrical and "concertina-like" configuration. This allows, for example, the socket to be expandable and compressible. Vent hols may be formed in the flutes.

The socket may be flexible. The flexibility may be provided by material choice and/or structural form.

In some embodiments the frame can be tensioned on to or around the socket. The frame may be relatively rigid and the socket may be relatively flexible so that tightening of the frame can tension the socket to provide a good fit onto the patient.

The frame may be attachment points for a removable cover.

Different aspects and embodiments of the invention may be used separately or together.

Further particular and preferred aspects of the present invention are set out in the accompanying independent and dependent claims. Features of the dependent claims may be combined with the features of the independent claims as appropriate, and in combination other than those explicitly set out in the claims.

Brief Description of the Drawings

The present invention will now be more particularly described, by way of example, with reference to the accompanying drawings, in which:

Figure 1 - exploded view of a prosthetic arm formed according to an embodiment.

Figure 2 - Palmar view of the tendon layout for a three-motor variant of an actuator block.

Figure 3 - Palmar view of the tendon layout for a four-motor variant of an actuator block.

Figure 4 - construction of a finger.

Figure 5 - A lever effect magnifies lateral forces at the joint.

Figure 6 - 2nd moment area of a rectangle

Figure 7 - simplified cross-section of the finger, showing the tendon path (highlighted in blue)

Figure 8 - cross-section of a flexed finger, showing the tendon path (highlighted in blue).

Figure 9 - The spring locations, highlighted in grey, in the 'knuckle' and 'distal' joint positions.

Figure 10 - The Brass Tendon Termination Barrel.

Figure 11 - Tendon wrapping and clamping.

Figure 12 - Tendon Termination in the Distal Phalanx.

Figure 13 - Alternative Tendon Tensioning/Clamping.

Figure 14 - Lateral cross section of the palm chassis and thumb ligament cover showing the path of the thumb tendon (highlighted).

Figure 15 - Exploded view of the palm.

Figure 16 - Left: Three motor variant. Right: Four motor variant. Centre: Annotated lateral view.

Figure 17A-Hitchhiker System.

Figure 17B - Normal Extension and Impeded Extension in a Hitchhiker system.

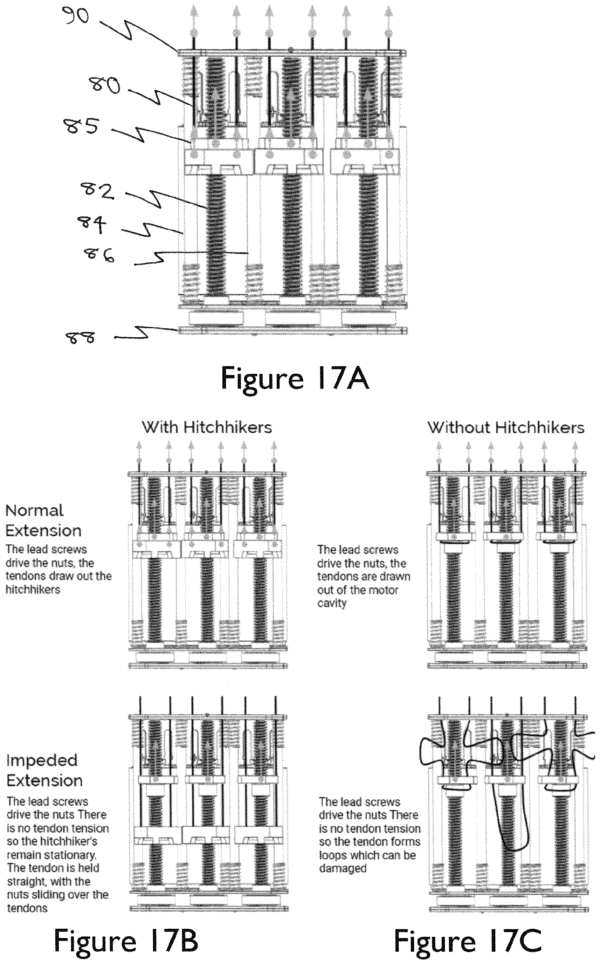

Figure 17B - Normal Extension and Impeded Extension in a System without Hitchhikers.

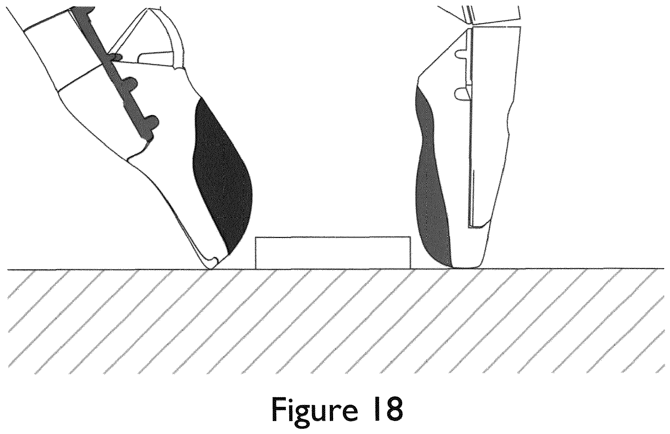

Figure 18 - A pinch grip around a coin. The need for grip pads to extend onto the distal end of digits.

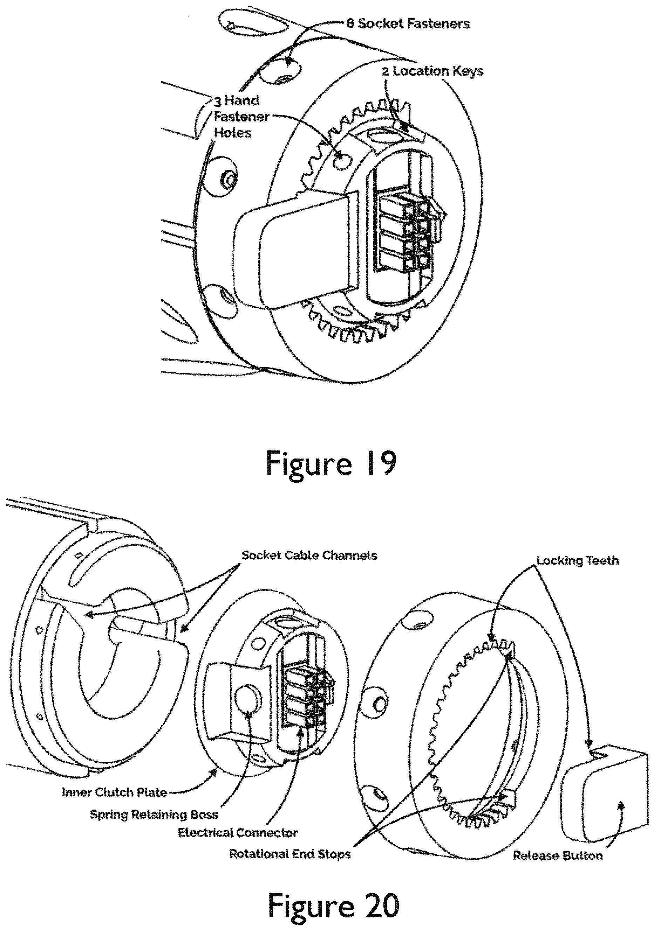

Figure 19 - wrist attachment method.

Figure 20 - Sub-components of the wrist mechanism.

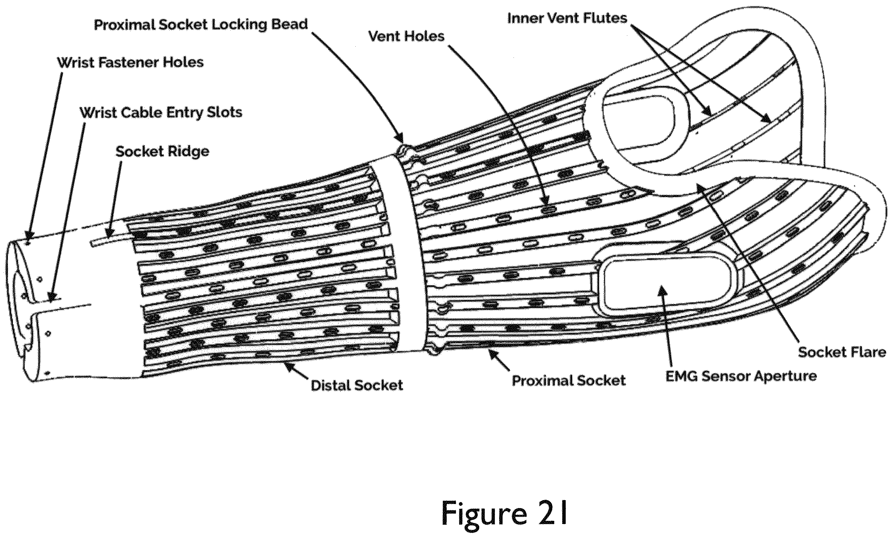

Figure 21 - Socket Nomenclature.

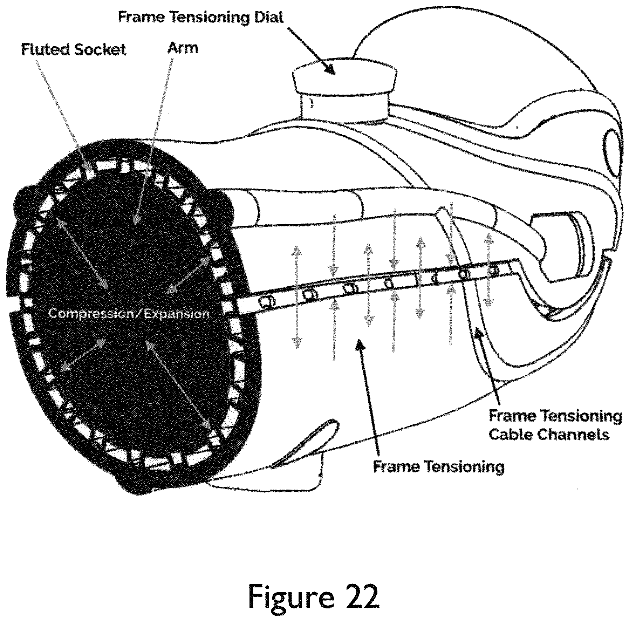

Figure 22 - Fluted Socket and Tensionable Frames.

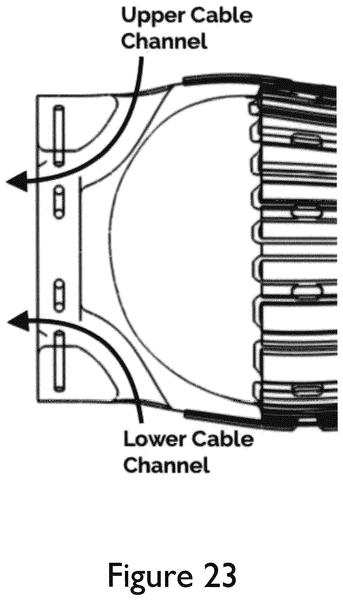

Figure 23 - Cable entry/exit channels.

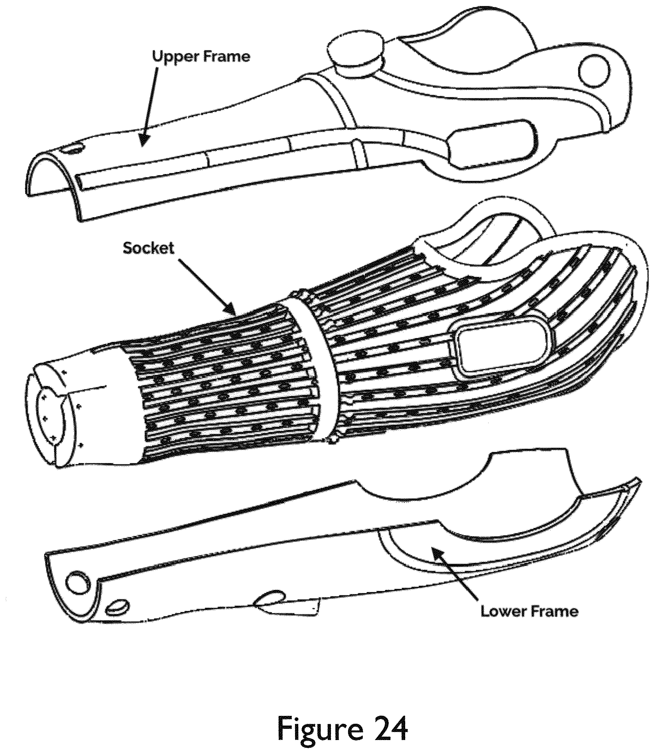

Figure 24 - Upper and Lower Clamping Frame Configuration.

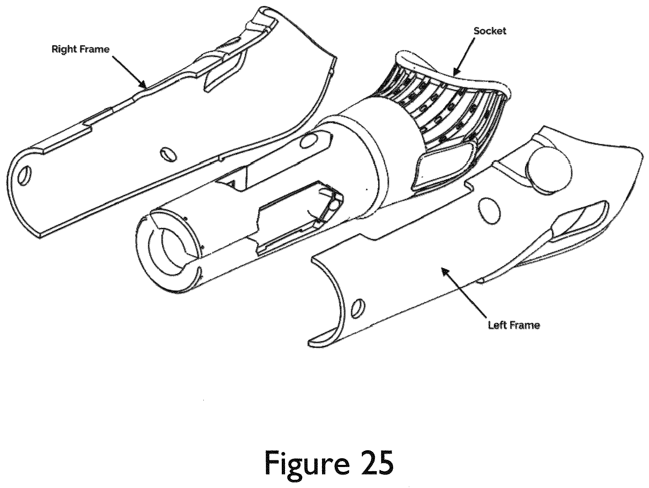

Figure 25 - Left and Right Clamping Frame Configuration.

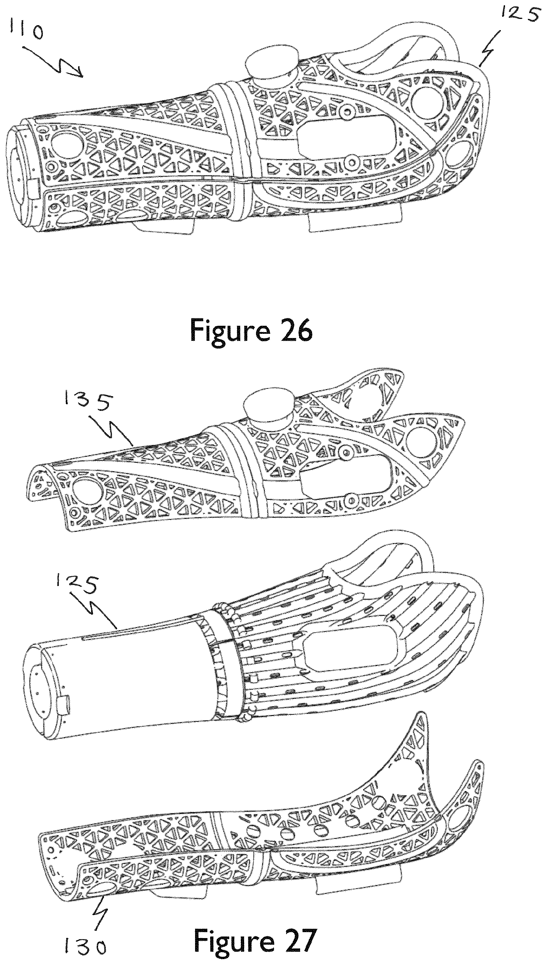

Figure 26 - Prosthetic Arm.

Figure 27 - Prosthetic Arm - exploded view.

Definitions

Palmar - the side of something closest to the palm.
Axial Plane - the plane defined by a normal running axial to the object in question. If no object is specified, it should be assumed the term is being used in the broader anatomical way where the axial vector runs from head to foot through the body.
CoM - Centre of Mass
$G^2$ - Geometric continuity in the 2nd derivative. Two curves, meet at a point, share a tangent and curvature.
DFMEA - Design Failure Modes Effects Analysis
PCB - Printed Circuit Board

Example embodiments are described below in sufficient detail to enable those of ordinary skill in the art to embody and implement the systems and processes herein described. It is important to understand that embodiments can be provided in many alternate forms and should not be construed as limited to the examples set forth herein.

Accordingly, while embodiments can be modified in various ways and take on various alternative forms, specific embodiments thereof are shown in the drawings and described in detail below as examples. There is no intent to limit to the particular forms disclosed. On the contrary, all modifications, equivalents, and alternatives falling within the scope of the appended claims should be included. Elements of the example embodiments are consistently denoted by the same reference numerals throughout the drawings and detailed description where appropriate.

The terminology used herein to describe embodiments is not intended to limit the scope. The articles "a," "an," and "the" are singular in that they have a single referent, however the use of the singular form in the present document should not preclude the presence of more than one referent. In other words, elements referred to in the singular can number one or more, unless the context clearly indicates otherwise. It will be further understood that the terms "comprises," "comprising," "includes," and/or "including," when used herein, specify the presence of stated features, items, steps, operations, elements, and/or components, but do not preclude the presence or addition of one or more other features, items, steps, operations, elements, components, and/or groups thereof.

Unless otherwise defined, all terms (including technical and scientific terms) used herein are to be interpreted as is customary in the art. It will be further understood that terms in common usage should also be interpreted as is customary in the relevant art and not in an idealized or overly formal sense unless expressly so defined herein.

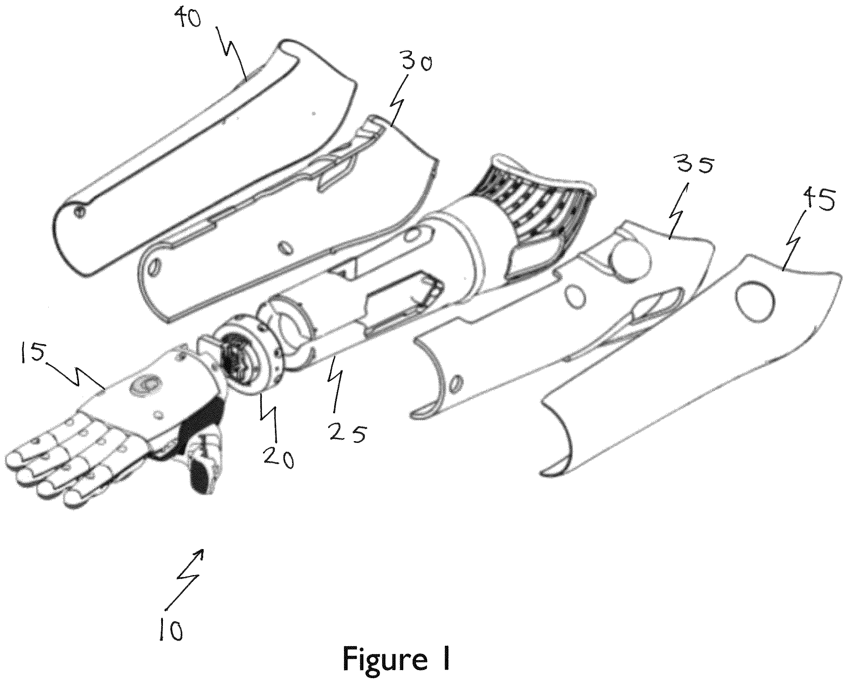

Referring first to Figure 1, there is shown a prosthetic arm 10 designed to fit transradial amputees. It is comprised of three main sub-assemblies; the hand 15; the wrist 20; and the socket 25. An outer frame is provided by two frame portions 30, 35. Additionally, optional, swappable covers 40, 45 can be added to style the arm and are detachably attachable to the outer frame portions.

The system is actuated by motors concealed within the palm. It is powered by a battery located either just below the elbow or inside the distal end of the arm. The user controls the system by flexing the muscles of their forearm; the system senses these flexes with Electromyographic (EMG) sensors embedded in the socket.

The arm is designed to offer amputees a level of functionality close to more advanced devices such as the BeBionic v3 from Otto Bock and the i-Limb from Touch Bionics, whilst still being affordable.

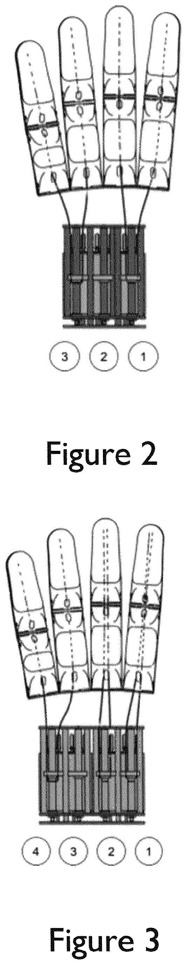

Figure 2 shows a palmar view of the tendon layout for the three motor variant of the actuator block. The central motor is linked to the thumb, this is omitted from this diagram.

The hand contains the actuators and the main control PCB. Although this places a large proportion of the mass far from the elbow, it means the hand can be fitted to a wide range of transradial amputees. Any hardware placed between the end of the user's residual limb and the wrist limits the range of residual limbs that can be fitted. Amputees with an intact wrist would have a disproportionately long prosthetic arm.

In this embodiment the humanoid hand has four fingers and a thumb. It comes in left and right variants, and a variety of sizes.

A smaller size variant uses a three motor actuator block. In this arrangement, the outer two motors are used to flex the fingers by pulling on a tendon.

A full description of the two actuator block variants is given below. In this arrangement, the outer two motors are used to flex the fingers. They do so by pulling on a tendon, a full description of this mechanism can be found below.

Motor one flexes the first and second fingers, motor two flexes the thumb, and motor three flexes the third and fourth fingers.

Figure 3 shows a palmar view of the tendon layout for a four motor variant of the actuator block. The third motor is linked to the thumb, this is omitted from the diagram.

For larger hands, there is space to fit a four motor variant of the actuator block. In this case, the first and second fingers are actuated independently. Motor one flexes the first finger, motor two the second finger, motor three is linked to the thumb, and motor four flexes the third and fourth fingers. The arrangement is shown in figure 3.

In this manner, hands with the four motor variant are capable of more dexterous grip patterns such as pinching.

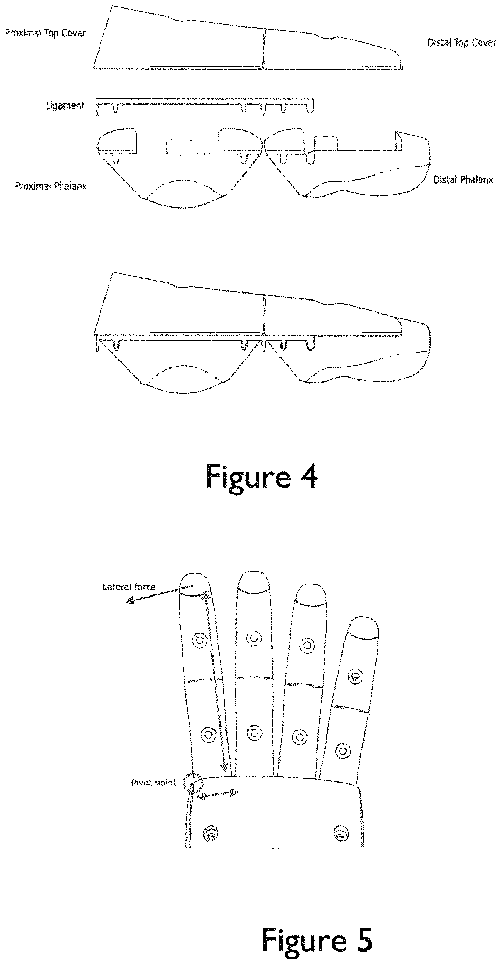

The fingers consist of a flexible "ligament" sandwiched between rigid phalanges and covers. An exploded view is shown in Figure 4.

The flexible ligament acts as a 'living hinge' between the rigid sections formed by the phalanges and covers. This form of joint was chosen because of its high durability. Due to their position on the distal end of the system, the fingers are likely to be the point of contact in any accidental collisions. They are also vulnerable to lateral forces as they are long compared to the width of their base; essentially a lever effect generates high forces at the joints.

The rubber 'living hinge' affords some flexibility, reducing the peak loads experienced in an impact. This also has a knock-on benefit for weight and weight-distribution. Less material is needed to reinforce each finger joint. The ligament is printed from Cheetah, an extremely abrasion resistant TPU. It is the same material the socket is made from. It is 3D printed flat to the print bed. In this way the layer lines, which are natural fault lines, are never loaded in shear or tension as the joint flexes. Additionally, the ligament is very quick to print in this orientation. The shape of the ligament at each joint is used to control the stiffness of the finger. At each joint, the axial cross-section of the ligament is comprised of two rectangles. The stiffness of a bending member of a given material is governed by a property of the cross-section called the 2nd Moment of Area. For a rectangle such as that seen in the ligament, the 2nd Moment of area is given by the equation:

$$I_x = \frac{b.h^3}{12}$$

Where $I_x$ is the 2nd Moment of Area, b and h are the width and height of the cross section respectively, and the cross-section is being bent around the x axis. By varying the values of b and h, the stiffness of the joints can be manipulated. The lateral stiffness is maximised by putting the bulk of the ligament at the edges of each finger. The stiffness against flexion was minimised by reducing b as much as possible. Beyond a certain point, the reduction of total cross section area means that snapping the ligament in tension when the finger is under load becomes a concern. The order in which the joints close can also be controlled by having different thicknesses at each joint. It is favourable for the distal joints to close after the proximal so the fingertips of the 1st and 2nd fingers meet the thumb in a pinch or tripod grip.

Actuation

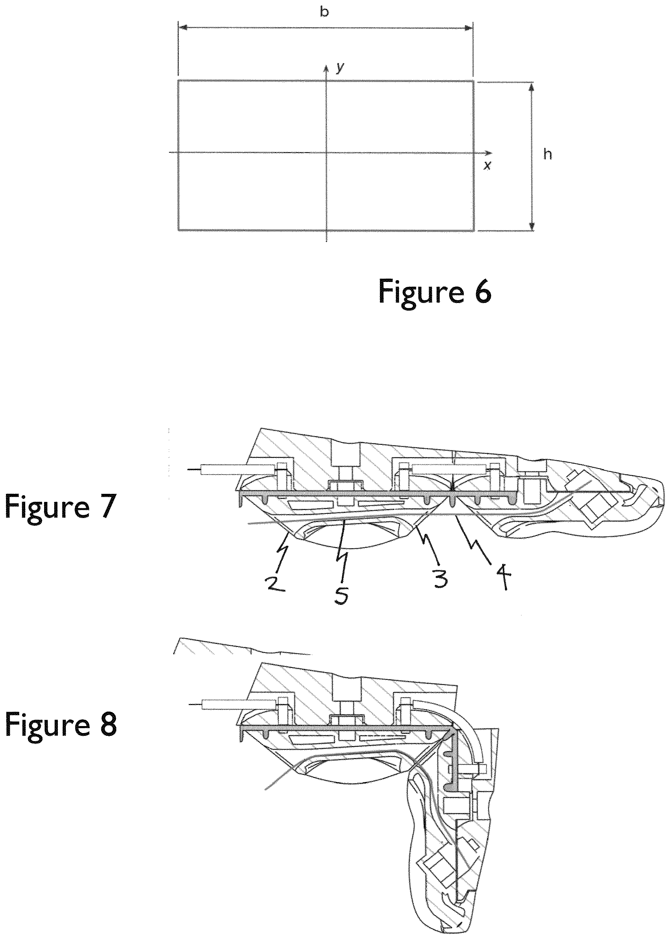

The fingers are flexed by means of tendons that run through tendon channels in the phalanges.
As the tendons are on the palmar side of the joints, the effect of the actuators retracting the tendons is to flex the fingers - Figure 7.
The magnitude of the flexing moment applied by the tendon at each joint is proportional to its perpendicular distance to it - Figure 8.

Joint Centre

Tendon Tension

Flexion Moment

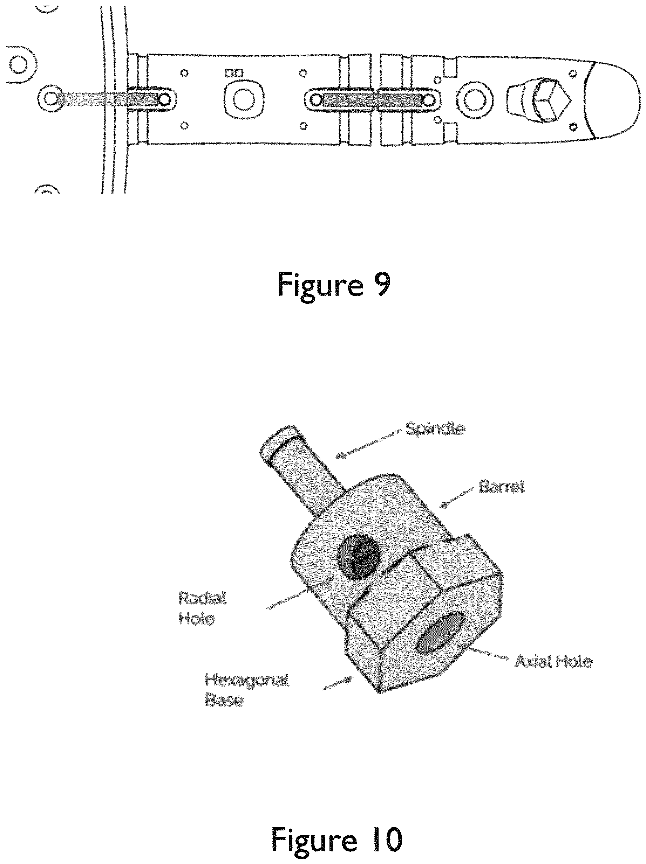

Lever arm length, x , with which tension in the tendon creates a flexion moment.
By moving the tendon away from the joint centre, a larger moment is generated for the same tension in tendon. The tension is related to the strength of the actuator. As this is essentially a question of leverage, increasing the perpendicular distance also increases the distance a tendon has to be retracted to fully flex a joint. The actuators in the hand produce linear motion through a lead screw. They therefore have a limited amount of travel. Therefore, the tendon channels have to be carefully positioned so that the full travel of the motor is used.
However, there is a further complication. Using the simple layout shown in Figure 9 causes very high pressures at the mouths of the tendon channels. When the joint is flexed, the tendon is bent through a large angle around a small radius, resulting in a very high normal force. This can produce a 'cheese-wire' effect leading to notches around the tendon channel mouth. The notches add friction and effectively change the perpendicular distance to the joint affecting travel. In order to prevent this the mouths 2, 3 of the tendon channel 5 are rounded, spreading the normal force over a larger area, reducing the pressure. This can be seen in Figure 7 and Figure 8.
The profile of the curve assumed by the tendon is an arc which is tangent to the palmar edge of the tendon channel and perpendicular to the joint face. The curves on either side of a joint also have the same radius. The perpendicularity and radius ensures the tendon is wrapped over a smooth (G 2 smooth) curve. The curvature also moves the tendon 4 away from the joint as the finger flexes, increasing leverage. This helps to counteract the increasing extension moments caused by the ligament and the springs.

Extension Springs

As the fingers are linked to the actuators via a flexible tendon, they are only flexed by the tendon retraction, and cannot extend via pushing of the tendon. To compensate for this, the fingers have a series of springs on the dorsal side which allows them to extend when the actuators relax the tendon. The springs have a slight pre-tension on them when the fingers are fully extended which holds them against their backstops. This prevents the fingers flexing under their own momentum as the user moves the hand around. Without the springs, users report a "floppy" feel to the fingers.
At the distal end, the spring is looped over a dowel pin which is closed off with the distal top cover to ensure the spring cannot slip off or pull free, this can be seen in Figure 7 and Figure 8. This simple method was chosen because it is very space efficient, and room is limited in the fingertip. The springs are only ever placed under the loads of its own spring force; therefore this anchor does not need to take a high load. At the proximal end the springs are secured by an M2 x 5 mm bolt that screws straight into the finger ligament cover. The springs used are a set size and spring rate. This allows for the lengths to be adjusted to provide the required pretension at each joint on the fingers. The pretension is set such that, during flexion, the knuckle joint bends before the distal to allow a more natural and practical grip position. These pretension lengths are kept consistent across the finger sizes/lengths.

Tendon Fastening

As shown in Figure 2 and Figure 3, the tendons are fastened at the fingertips. The tendons need to be exactly the right length for each finger. Otherwise, some of the travel would be used taking in slack, or the finger would not be able to fully extend. The tendons are also under extremely high loads. Due to the leverage involved in a tendon system (see Figure 9), the tension in the tendon can be an order of magnitude higher than the grip force exerted by a fingertip. As such, the tendon fastening mechanism needs to be both adjustable and capable of withstanding high loads. It also needs to be compact enough to fit in the fingertip.

Several approaches were tried. Simply tying a terminal knot, such as those used to tie a fishing hook to a line, is an extremely space efficient solution. However, it's extremely hard to get the tendon length correct. Clamping the tendon in a printed cleat was experimented with. Due to its small diameter (about a quarter of a millimeter), the tendon can simply notch the printed parts and work loose.

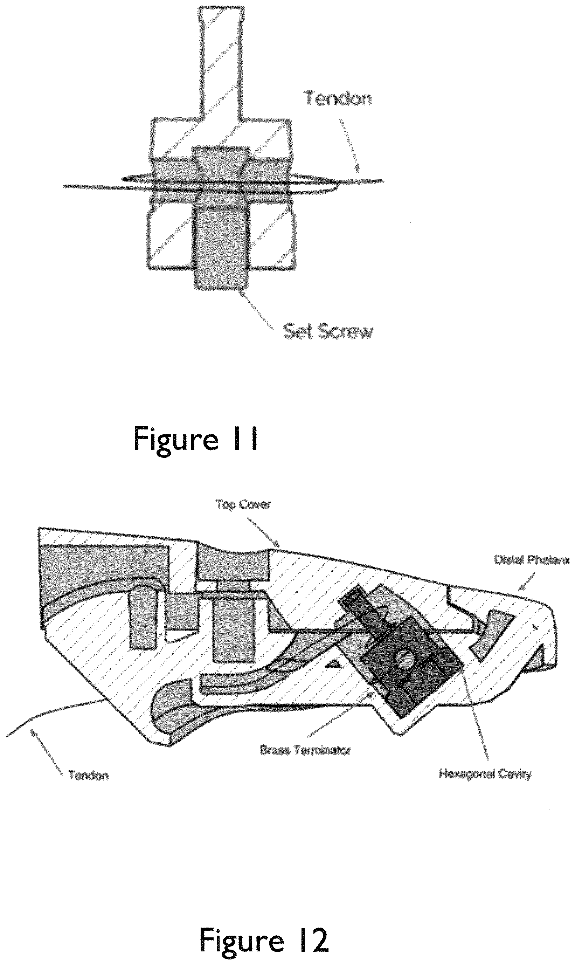

Wrapping the tendon around a bolt anticlockwise (so that tension in the tendon does not work to loosen the bolt) and then tightening the bolt was also explored. This approach presented two issues; slip of the tendon, and repeat tightening/loosening of the bolt during assembling causing damage to the tendon. An FDM printed ratcheting mechanism was also explored, and whilst it eliminated the issues of tendon damage and slip, the extremely high loads the tendons can be exposed to caused the printed part to shear. To overcome these numerous issues, a design was chosen whereby the tendon is fastened to a machined brass barrel comprised of: a hexagonal base for positional control; a radial hole for threading the Tendon through; an axial hole for clamping the Tendon and a narrow spindle around which the Tendon is wrapped, as shown in Figure 11.

To mitigate the risk of slip, and eliminate variation inherent in terminating the Tendon with a knot, the tendon is inserted through a hole in the brass barrel, wrapped around it, and then inserted through the same hole again - this loop of Tendon is then clamped in place using a set screw with a flat top (to avoid the risk of the screw damaging the tendon), as shown in Figure 12.

The hexagonal base of the brass barrel is inserted into a matching cavity in each of the fingertips and thumb, as shown in Figure 12, allowing for the barrel to sit in one of six positions. Thus the tendon can be tightened to the correct length by incrementing the rotation of the barrel, and hence causing additional tendon length to wrap around the spindle.

The possible increment of the tendon is given by:

$$\Delta L_{Tendon} = \frac{\pi.D_{Spindle}}{n}$$

Where,

$\Delta L_{Tendon}$ is the change in Length of the Tendon
D is the Diameter the Spindle
n is the Number of Sides on the base of the barrel

Therefore

$$\Delta L_{Tendon} = \frac{\pi.1.5}{6} = 0.52mm$$

Further fine tuning of the tendons once the product is assembled can be achieved with motor calibration. Motor calibration is described below.

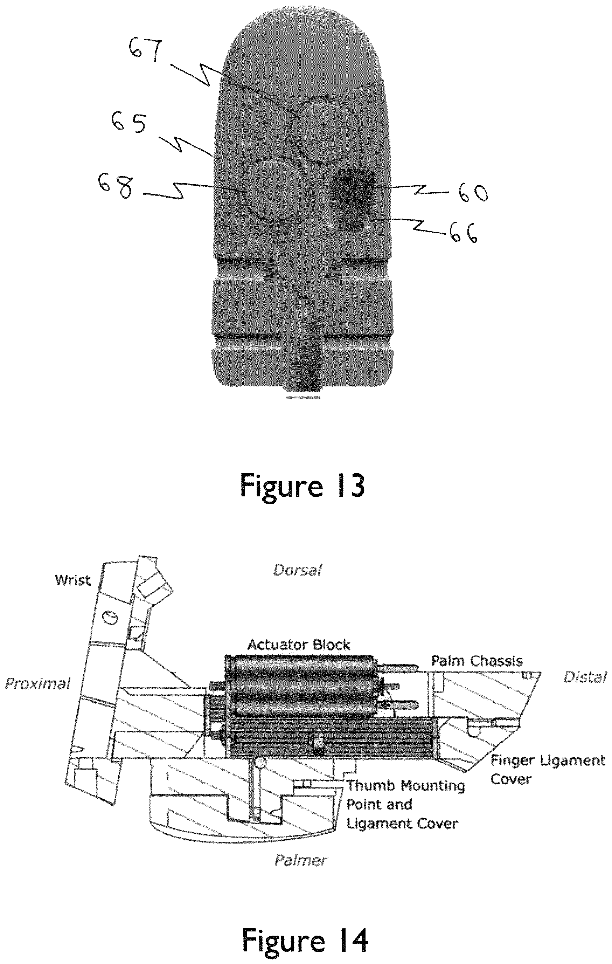

Figure 13 shows a tendon fastening system used in some embodiments. The tendon 60 exits the fingertip 65 from a channel outlet port 66. Two large-headed bolts 67, 68 are provided. The tendon can be wound around the bolts in a figure of eight configuration as shown. The tendon can be slid through to change the effective working length. To secure the tendon the bolt heads are screwed down.

Thumb

The thumb tendon exits through a hole in the thumb mounting point. A consequence of this is that the thumb motor is reversed compared to the finger motors. The nut moves distally (towards the fingers) to oppose the thumb.

Due to the required path of the tendon, there is a lateral component to the tensional force it applies to the nut and lead screw. It is not always axial as is the case in the finger tendons. The lateral force reduces as the thumb is opposed. This is because the nut is further towards the distal end of the travel and the angle between tendon and the lead screw is reduced. There is a small risk of the lead screw being deformed if the thumb is shock loaded when there is a high lateral component to the tendon tension. However, the cheetah ligaments provide shock absorption to mitigate this. Additionally, the thumb will most likely hit the backstop if subject to a shock load in the open direction when fully open (unopposed). The force would then not be transmitted through the tendon to the lead screw.

There is a passive (non-powered) distal joint on the thumb. This is designed to allow it to fold flat to the body of the palm, to aid with the donning and doffing of clothing. Without this feature, the thumb protrudes sufficiently to prevent the prosthesis from being passed down a sleeve.

Previously, the thumb had two actuated joints in a similar manner to the fingers. However, this can cause a problem referred to as "buckling". Essentially, when the hand was used to pinch objects, the proximal joint was fully flexed, and the distal extended. As the joints were actuated by the same tendon, this meant the motor was approximately halfway along its travel. The problem occurred when a load was applied to the tip of the thumb, the proximal joint could extend if the distal joint flexed, thus moving the tip of the thumb away from the first finger, completely preventing a pinch.

Making the distal joint active, and the proximal passive was also considered. This enables a "key" grip where the thumb, in an unopposed position, closes against the side of a fully flexed first finger. The actuated proximal joint was chosen because it was thought that users would change grip more times than they would don or doff clothing. Therefore, they would need to intervene to manipulate the passive joint less frequently.

The passive distal joint is of very similar construction to finger joints. However, it has the additional of clicking between the flexed and extended positions. There is a cylindrical protrusion on the proximal phalanx. Its axis is collinear with rotation axis of the joint. It extends between the two sides of the ligament. The ligament has a 0.2 mm interference with the cylinder meaning the inside edges sweep the flat surfaces as the joint flexes or extends. At the limits of travel of the joint, there are two grooves for the ligament to click into.

The thumb can oppose far enough that it is directly between fingers three and four (middle and ring). It moves to this position in a full fist grip so it opposes all four fingers equally. For tripod grip, it opposes to the point where it is between fingers one and two. For pinch grip, it meets the first finger. This positioning is done to create stable grips. If the thumb does not oppose all involved digits equally, a torque is applied to the payload object.

Palm

The palm of the hand contains the control PCB, and the actuators.
The central component of the hand is the chassis. It is the main load bearing component of the palm. It also provides attachment points for the finger ligament, knuckle, the actuator and PCB block, the thumb and the wrist.

The main load path through the hand goes from the fingers, through the chassis, and finally the wrist. The chassis and wrist dovetail together to provide a strong connection, which is reinforced by two M2 bolts. The wrist and chassis are printed as separate parts to ensure the layer lines are oriented favourably for both components.

The grip pad is cast from a soft (Shore Hardness A 30) urethane. This allows it to deform around payloads, thus providing a more stable grip. In previous iterations, we have tried printing the palm as part of the chassis from a soft TPU. However, as 3D printing filaments have to be pushed through a nozzle by an extruder gear, there is a limit to how soft they can be. By printing slowly on a modified printer, some success was had printing materials as soft as Shore A 60. However, casting in 3D printed moulds is more scalable for production. The urethane grip pads are cast, onto the "palm frame" component. The honeycomb internal structure of the 3D printed frame is exposed on several faces to allow urethane to flow in and achieve a good bond. The frame then bolts to the dorsal cover, the two components sandwiching the chassis and internal components. This makes it easy to replace in the event the grip pad is damaged. When these two components are removed, there is clear access to the PCB, and the underside of the motors. This is useful for maintenance.

The dorsal cover is a 2 mm thick shell that leaves an air gap around the motors and PCB. This component is designed in a way that reduces weight.

The finger ligament cover clamps the finger ligament tightly to improve torsional and lateral stiffness. It also contains the bolts described below for fastening the finger extension springs.

The dorsal cover, knuckle, and wrist components all have interlocking lip features between them to prevent the ingress of water and dirt. The prosthesis is thereby IP33 rated.

The button cover is cast from Dragon Skin 30, Translucent Platinum Silicone which is easy to work with, picks up detail well and is skin safe. It is cast in a high resolution (0.06 mm layer height), 3D printed mould. The circle design and logo relief encourages users to push the centre of the button. The stiffness of the silicone and the better support around the edge of the button has also provided a more reliable off-centre button push response. A wide lip around the button acts as a seal against the dorsal cover, reducing the chance of ingress of dirt or liquid which could damage the internals. The SD card support piece was designed to sit under the button cover and the PCB's SD card to act as a support for both and to protect the PCB components from load stresses during button push.

Internal Layout

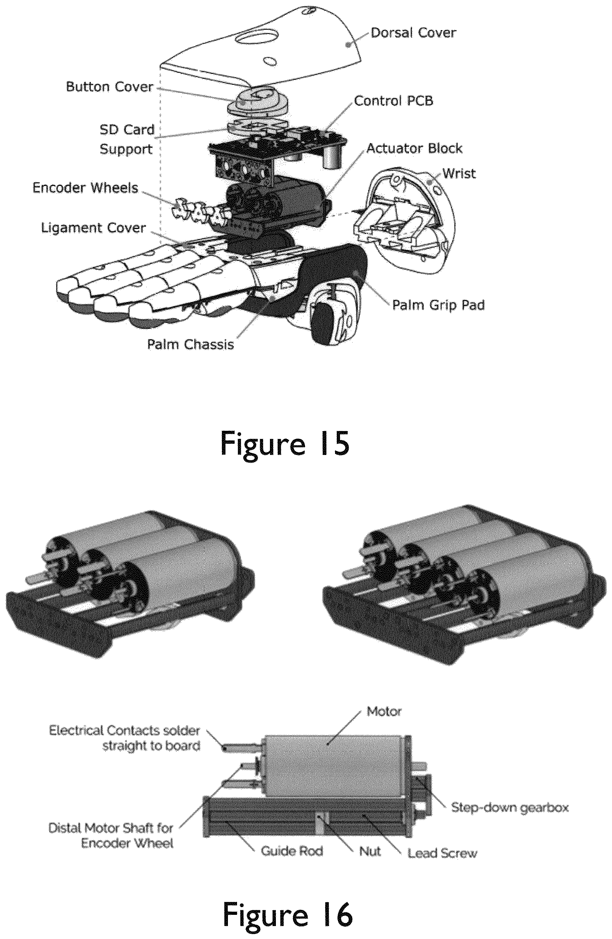

The actuators are comprised of DCX12L motors and a custom gearbox, both provided by Maxon Motor. The gearbox steps down the motor output and converts it from rotational movement to a linear movement via a lead screw. The motor is situated above the lead screw as shown in figure 15.

This arrangement allows the nut on the lead screw to travel the full distance of the unit. This maximises the energy (i.e. force x distance) available to flex the fingers.

The position of the nuts is measured by rotary encoders attached to the distal end of the motors. As encoders measure movement, rather than the absolute position of the nuts on the lead screws, the ability to detect when the nuts are at the ends of the lead screw is required. This is done by slowly extending the motors until the speed of encoder clicks drops off as the nut hits the compression springs at the end of its travel. The springs also prevent high shock loads in the case of a nut hitting its endstop. The PCB was designed to fit around the motors as compactly as possible, and to fit in the natural shape of the hand. As such, all the tall components are along the central axis of the dorsal side of the board, with the exception of the two large power capacitors that are on the palmar side behind the motors. The rest of the palmar side has low profile parts allowing it to fit as close to the motors as possible.

Figure 17A illustrates the functionality of the actuator block.

The motors work through a gearbox to move drive nuts backwards and forwards along lead screws.

Each tendon 80 is connected to a drive nut 85 (e.g. the nut has two holes and the tendon is threaded through the holes) so the tendon moves with the nut. The nut is threaded onto a lead screw 82. Running parallel to and either side of the lead screw is a guide rod 84, 86. The nut is prevented from rotating by the guide rods, along which the nut can slide. This means that when the lead screw 82 is rotated by the motor, depending on which way the screw is rotated, the nut moves towards the proximal end 88 or the distal end 90 of the block.

When the nut moves towards the proximal end this pulls on the tendon and the finger will flex. When the nut moves towards the distal end this relaxes the tendon and the springs cause the finger to straighten (i.e. the motors do not power finger extension).

In normal operation the tendon will move backwards and forwards with the nut, with no slack in the tendon. However, as illustrated in Figure 17C, if extension of the finger is impeded (e.g. if the user tries to straighten the finger but cannot, for example because they are holding it closed, or if the fingers are prevented from straightening as they are held on a surface) this can create a problem. For example, if the finger is fully flexed, with the nut at or towards the proximal ends, and then the user tries to straighten the finger (but cannot) there is no tendon tension; this causes slack in the tendon.

To solve this problem the present invention conceives of the use of "hitchhikers" 74. The hitchhikers 74 are not attached to the lead screw, but they can slide along the guide rods 74, 76. The tendon is also looped through the hitchhikers.

In normal operation the hitchhiker is pushed proximally by the nut and is pulled by the tendon distally, so that it follows the nut. For example as the finger is bent the nut is moved proximally by the motor and the hitchhiker is pushed proximally by the nut. As the finger straightens the nut is moved distally by the motor and the hitchhiker is pulled by the tendon (and is allowed to move along behind the nut).

In the case where the finger cannot straighten the hitchhiker stays where it is even though the nut is being moved by the motor. There is no tendon tension so the hitchhiker remains stationary, preventing slack in the tendon. When the finger is eventually released there is tension back on the tendon and the hitchhiker is pulled back onto the proximal side of the nut.

Figures 17B and 17C illustrates the functionality of the actuator block and also a "hitchhiker" system used in some aspects and embodiments of the present invention.

Grip Pads

The grip pads on the palm, fingers and thumb are cast from a urethane rubber. Shore Hardness A 30 was chosen as a balance between robustness and ability to deform around payloads. The grip pads are all cast onto PLA components that are designed to be hollow. The printed parts are placed in the mould for the fingers, and the urethane is cast directly onto the final part. The urethane floods the internal cavity of the parts, engulfing the internal features such as the tendon channels in the case of the fingers. The mechanical purchase, large contact area, and absence of any exposed peel-edges produces a strong bond. The grip pads need cover the parts of the hand that make contact with payloads. In the case of the distal phalanges, it is important that the grip pad extends as far around the fingertip as possible. This allows the hand to pick up low objects from tables, for example a coin. This is shown in Figure 18.

As the urethane needs a lip around its edge to prevent peeling, the distal phalanges have a cavity at their tip that extends dorsally of the cover split line. The distal cover locks into a socket at the proximal side of this protrusion. The protrusion also gives the impression of a fingernail.

The shape of the grip pads is based on the shape of a human finger. They're convex meaning that when they make contact with an object, they depress inwards and the surrounding material is also pressed onto the payload. The increase surface area of the rubbery material improves the grip. The dorsal side of the digits square off slightly to help with the scenario shown in Figure 18.

Wrist

Attachment Interfaces

Figure 19 shows the attachment of the hand to the wrist mechanism, and is semi-permanent via three screws radially positioned. The screws can be removed along with the hand for maintenance.

The radial torque from the socket to the hand is transmitted via two keys so that the radial screws are disassociated and are just providing a pull-off constraint putting the screws in shear which is their strongest property.

Attachment of the wrist to the socket is via eight radial self-tapping fasteners that screw into the Cheetah based material of the socket liner, again the screws are in shear which is utilising their strongest property to resist pull-off loads. The Cheetah material is semi flexible and will heavily resist vibration related unfastening.

Rotating Mechanism

Figure 20 shows the sub-components of the wrist mechanism. The wrist has been designed to rotate the hand +/-90 deg ° from a neutral position. The neutral position has been defined as the hand in the vertical plane with the thumb upwards. Therefore the hand can be indexed to the palm up or palm down position.

The wrist rotation is naturally locked with a button on the dorsal side of the wrist requiring to be depressed to unlock. Depression of the button releases internal gear teeth allowing indexing of the hand at approximately 7° increments. A spring forces the teeth on the button back into place locking the wrist upon release. There are different (for example two) sizes of wrist diameters, each use the same internal components and mechanism, only the outside diameter and release button length is modified.

Cable Management

The wrist has to allow pass through of both power and EMG signal cables between the hand and the socket components. Because the locking and index mechanism is low profile, an 8-pin connector has been incorporated into the central space, which connects to the hand's main board upon hand fitment. Behind this connector, there is space for a spiral wrap of cables which will expand and contract as the wrist rotates. At the distal end of the socket, the cables will split into two different feeds, one for the EMG circuit on and one for the battery pack. The length of the wrist section is 20mm and diameters are, for example, 56mm (large) and 46mm (small).

Arm

Socket

In this embodiment the socket/liner is printed in the semi flexible Cheetah plastic from Fenner Drives which is a certified medical safe material to ISO 10993, tested by Envigo Laboratory.

Due to the socket's flexibility and design profile, it is both expandable and compressible which allows some growing room and an element of conformality to the user's residual arm shape.

Adjustability of the fit comes from the external panels compressing on the outer surface of the socket via a cable tensioning system.

Figure 21 shows the various features on the socket. Ventilation is achieved due to the fluted nature of the socket where small air channels have been incorporated, the fluted channels are printed with holes to allow heat and moisture to escape externally and allow fresh air to permeate through to the skin. The covering frames are also aerated via a mesh like structure which helps reduce heat containment.

Figure 22 shows how the covering frames compress on the flutes via a tensioning system and due to the thin walled nature of the flutes, the socket adjusts its diameter to conform to a range of shapes.

The socket is printed in two parts, a fixed distal section which is attached to the wrist via the eight socket fasteners described in Figure 19 and a removable proximal section that can be washed and cleaned easily.

The double section socket also makes the 3D printing process of some embodiments more stable by reducing the need to print tall slender flexible objects. The proximal section of the socket is held in place by a locking bead feature which is captivated by the outer frames coupled with a cable tensioning system.

For each patient the optimum EMG sensor position should be attained. The socket has cutouts shaped so that sensor assembly can be pushed through from the outside to achieve fitment against the skin at the desired location.

Cable management has to pass through from the outside of the arm, through the outer frames and socket into the wrist. The distal end of the socket has channels for the EMG and battery power cables to pass through as shown in Figure 23.

The flared entry around the elbow has been extended to cover the epicondyle areas to achieve some clamp and prevent the socket from falling off. During the scan rectification phase, these areas can be reworked to give extra clamp. These areas on the clamping frames can also be reworked with heat at the patient fitment phase. Running along the length of the socket are location ridges for the outer frames, this is to stop any radial slip during the tightening process with a cable tensioning system.

Thermoformed Frames

In some embodiments, external to the socket are two large frames that can be used to provide an adjustable clamping force to retain the socket on the arm.

Two example configurations of the arm, which impacts the shape of the frames, are shown.

One configuration is to have the battery pack attached externally to the arm and for this we split the frames into an upper and lower configuration (Figure 24). The second configuration is to have the battery internal to the distal end of the arm and for this we split the frame into a left and right configuration (Figure 25).

The frames are attached to the distal end of the socket by four self-tapping fasteners in each frame. Figures 26 and 27 show various features on a prosthetic arm 110 formed in accordance with the present invention. The socket 125 is shown and external to the socket are two large frames 130, 135 that provide an adjustable clamping force to retain the socket on the arm.

In some embodiments the frames are designed to be 3D printed flat and then thermoformed with heat to their desired shape on a 3D printed This method achieves quick 3D print time and much stronger part due to the lamina direction. Forming a flat frame creates a lamina flow which follows the contours of the arm in a similar manner that a forged part creates a flowing grain direction that follows the shape of a component. To aid the thermoforming process, the forming buck is 3D printed, which is a copy of the patient's arm scan with some extra features. Location features are modelled into the buck to align important details in the frames such as EMG sensors, tensioner and cover attachment locations.

Another feature gained by FDM (Fused Deposition Modelling) printing flat is the ability to print the part without any upper and lower surfaces thus exposing the triangular mesh inside, this is known as "Open Core". It creates a very lightweight, yet strong and faster to print component that allows a good amount of airflow through the frame to the internal socket. 3D printing this mesh in this way removes the time and complexity of modelling the ventilation in CAD. The mesh allows a bit more stretch and compression when working the frame over the forming bucks during thermoforming, reducing the risk of creasing. Large flat frames are also split proximal into and distal sub-components to allow them to fit on a 3D printing bed. The proximal and distal frames are joined by a friction stir weld before forming. The location of the joint is subtly hidden in the distal socket locking bead groove. On the underside of the frames are longitudinal grooves which line up with the socket ridges to help alignment during the thermoform phase and tensioning the socket. In other embodiments the frames are formed in their final shape e.g. printed 3D by a selective laser sintering process.

Covers - Standard covers may be auto-generated as part of the CAD process. There are two configurations which match the frames, either an upper/lower or a left/right configuration for an external or internal battery respectively. The upper/lower configuration also has a short lower cover supplied to protect the battery only, this is to enable the user to wear the arm with very minimal covers to reduce weight and increase airflow cooling. The covers are attached by push fit pins that are printed integral to the covers at the distal end. The pins press into and are retained by a flexible Cheetah-based insert into the frames. At the proximal end of the covers there are two 3M branded Dual Lock pads affixed to the covers by glue. These pads locate with the similar pads attached to the frames and pressing them together forms a strong but removable bond. The short battery cover option does not have distal pins but four of the dual lock pads, one in each corner for fixation.

Material Choice

In some embodiments the printed parts are all made from two materials. The rigid parts are made from PLA, a biodegradable thermoplastic. PLA has been used in medical implant applications I. The specific PLA used in the OBI is produced by Filamentive. It is stated as being "essentially non-irritating to skin" in the Safety Data Sheet. No PLA parts are in prolonged contact with the skin so this is considered low risk. The flexible parts such as the ligaments and socket are made from a TPU designed for 3D printing called "Cheetah" made by a company called Ninja Tek, a subsidiary of Fenner Drives. Cheetah is nontoxic, and certified for long term use in contact with skin. The grip pads may be cast from Vytaflex 30 Urethane rubber.

Although illustrative embodiments of the invention have been disclosed in detail herein, with reference to the accompanying drawings, it is understood that the invention is not limited to the precise embodiments shown and that various changes and modifications can be effected therein by one skilled in the art without departing from the scope of the invention as defined by the appended claims and their equivalents.

**Claims**

1. A prosthetic digit comprising one or more channels through which tendon-like members can pass to cause flexing of a joint, the channel/s having a mouth at each end thereof, the mouths being rounded, whereby in use to spread the normal tendon force over a larger area, thereby reducing the pressure.

2. A digit as claimed in claim 1, in which the digits have phalanges and the channels pass through the phalanges.

3. A digit as claimed in claim 1 or claim 2, in which the tendon terminates in a tip region of the digit.

4. A digit as claimed in any preceding claim, in which the digit has means for adjusting the tension in the tendon.

5. A digit as claimed in any preceding claim the digit having phalanges which can flex via retraction of a tendon-like member.

6. A digit as claimed in any preceding claim, in which the finger having, on its dorsal sides, an extension spring for causing the finger to extend when the tendon is relaxed.

7. A prosthetic hand or foot having one or more digits as claimed in any preceding claim.

8. A hand or foot as claimed in claim 7, comprising one or more actuators for actuating digits.

9. A hand or foot as claimed in claim 7 or claim 8, comprising a main control PCB.

10. A hand as claimed in any of claims 7 to 9, in which the hand has four fingers and a thumb.

11. A prosthetic finger comprising a channel through which a tendon-like member can pass to cause flexing of a joint, the channel having a mouth at each end thereof, the mouths being rounded.

12. A finger as claimed in claim 11, in which more tendon is assigned to a proximal joint than to a distal joint, whereby the proximal joint flexes before the distal joint.

13. A finger as claimed in claim 11 or claim 12, in which the tendon is positioned towards the top of the channel when the finger is relaxed, and moved to the bottom of the channel when the finger is bent.

14. A finger as claimed in any of claims 11 to 13, in which in a bent position the tendon is constantly curved in an arc across the joint.

**15.** A finger as claimed in any of claims 11 to 14, in which the curves on either side of a joint also have the same radius as the mouths.

Figure 1

Figure 2

Figure 3

Proximal Top Cover

Distal Top Cover

Ligament

Proximal Phalanx

Distal Phalanx

# Figure 4

Lateral force

Pivot point

# Figure 5

## Figure 6

## Figure 7

## Figure 8

# Figure 9

Spindle

Barrel

Radial
Hole

Axial Hole

Hexagonal
Base

# Figure 10

Tendon

Set Screw

Figure 11

Top Cover

Distal Phalanx

Tendon

Brass Terminator

Hexagonal Cavity

Figure 12

67

65

68

60

66

## Figure 13

Wrist

*Dorsal*

Actuator Block

Palm Chassis

*Distal*

*Proximal*

Finger Ligament
Cover

Thumb Mounting
Point and
Ligament Cover

*Palmer*

## Figure 14

Dorsal Cover
Button Cover
Control PCB
SD Card Support
Actuator Block
Wrist
Encoder Wheels
Ligament Cover
Palm Grip Pad
Palm Chassis

Figure 15

Motor
Electrical Contacts solder straight to board
Step-down gearbox
Distal Motor Shaft for Encoder Wheel
Guide Rod    Nut    Lead Screw

Figure 16

Figure 17A

With Hitchhikers

Without Hitchhikers

Normal
Extension
The lead screws
drive the nuts, the
tendons draw out the
hitchhikers

The lead screws
drive the nuts, the
tendons are drawn
out of the motor
cavity

Impeded
Extension
The lead screws
drive the nuts There
is no tendon tension
so the hitchhiker's
remain stationary.
The tendon is held
straight, with the
nuts sliding over the
tendons

The lead screws
drive the nuts There
is no tendon tension
so the tendon forms
loops which can be
damaged

Figure 17B

Figure 17C

Figure 18

Figure 19

Figure 20

Figure 21

**Frame Tensioning Dial**

**Fluted Socket**   **Arm**

Compression/Expansion

**Frame Tensioning
Cable Channels**

**Frame Tensioning**

Figure 22

Figure 23

Upper Frame

Socket

Lower Frame

Figure 24

Right Frame

Socket

Left Frame

Figure 25

110

125

## Figure 26

135

125

130

## Figure 27

Europäisches
Patentamt

European
Patent Office

Office européen
des brevets

## EUROPEAN SEARCH REPORT

Application Number

EP 19 16 6427

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/005871 A1 (UNIV CAPE TOWN [ZA]) 14 January 2016 (2016-01-14) * page 6, line 31 - page 7, line 9 * * page 8, line 4 - page 9, line 4; figures 1, 15 * | 1-15 | INV. A61F2/56 A61F2/68 A61F2/58 |
| A | GB 2 488 760 A (STROVER ANGUS EVERETT [GB]) 12 September 2012 (2012-09-12) * figures 1, 2 * | 1-15 | |
| A | US 2006/224249 A1 (WINFREY REX C [US]) 5 October 2006 (2006-10-05) * figure 13 * | 1-15 | |

TECHNICAL FIELDS
SEARCHED (IPC)

A61F

The present search report has been drawn up for all claims

| Place of search | Date of completion of the search | Examiner |
|---|---|---|
| Munich | 21 August 2019 | Jansson Godoy, Nina |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 19 16 6427

This annex lists the patent family members relating to the patent documents cited in the above-mentioned European search report.
The members are as contained in the European Patent Office EDP file on
The European Patent Office is in no way liable for these particulars which are merely given for the purpose of information.

21-08-2019

| Patent document cited in search report | | Publication date | Patent family member(s) | | Publication date |
|---|---|---|---|---|---|
| WO 2016005871 | A1 | 14-01-2016 | GB<br>WO<br>ZA | 2528049 A<br>2016005871 A1<br>201608736 B | 13-01-2016<br>14-01-2016<br>27-09-2017 |
| GB 2488760 | A | 12-09-2012 | NONE | | |
| US 2006224249 | A1 | 05-10-2006 | NONE | | |

EPO FORM P0459

For more details about this annex : see Official Journal of the European Patent Office, No. 12/82